# EUROPEAN PATENT APPLICATION

(11) **EP 4 516 246 A1**
(43) Date of publication of application: **05.03.2025**
(21) Application number: 23194580.9
(22) Date of filing: 31.08.2023
(51) Int. Cl.: A61B 17/221, A61B 17/22

(54) **CLOT REMOVAL DEVICE**

(71) Applicant: Martínez-Galdámez Ruiz, Mario, 28231 Madrid (ES)
(72) Inventor: Martínez-Galdámez Ruiz, Mario, 28231 Madrid (ES)
(74) Representative: Icosa

(57) **Abstract**

The present invention relates to a clot removal device (100) for removing at least one clot (11) from an inside of a blood vessel (12) comprising a bifurcation (16), said clot removal device (100) comprising a clot removal member (31), configured to be positioned at the bifurcation (16), the clot removal member (31) comprising a base (32) configured to be positioned inside the proximal blood vessel (13), and two anchoring members (33, 34), wherein at least one of the anchoring members (33, 34) is configured to be positioned inside at least one of the two distal blood vessels (14, 15), each anchoring member (33, 34) comprising at least one self-expanding portion (35, 36) configured to adopt a collapsed configuration configured for navigating the clot removal device (100) inside said blood vessel (12), and an expanded configuration configured for anchoring to the clot (11).

## Description

### FIELD OF INVENTION

The present invention relates to a clot removal device for removing at least one clot from an inside of a blood vessel. The invention further relates to a system for clot removal comprising a clot removal device.

### BACKGROUND OF INVENTION

A blood clot, also known as a thrombus, is a gel-like mass of blood cells that seals off a damaged blood vessel and prevents further blood loss.

While blood clots are essential for preventing excessive bleeding, they can also pose risks if they form inappropriately or occur in critical blood vessels. Indeed, blood clots can be problematic if they obstruct blood flow or dislodge and travel to other parts of the body, leading to potentially life-threatening conditions. When a blood clot travels and lodges in a blood vessel, it is called an embolus, and the condition is known as an embolism.

Blood clots are naturally dissolved by the body through a process called fibrinolysis. To help this process, a patient may be given medications such as anticoagulant to prevent further clotting and promote the body's natural removal process. In some cases, especially when there is a severe or life-threatening clot, the use of a mechanical intervention may be necessary to remove or break down the clot.

Clot removal devices, also called "stentrievers" or "stent-retrievers", are mechanical devices specialized in endovascular procedures for clot retrieval, particularly in the treatment of acute ischemic strokes. Clot removal devices are configured to capture and remove the clot from a blocked blood vessel in the brain to restore blood flow.

To that end, a clot removal device has a tubular shape with a mesh-like structure comprising interconnected wires arranged in a pattern allowing for radial expansion near a blood clot.

There are several challenges associated with designing performant clot removal devices. Indeed, the vasculature of neurovascular vessels is even more fragile than other similar-sized vessels from other parts of the body. Excessive forces applied on these vessels can result in perforations and hemorrhages.

Furthermore, clot removal devices rely on outward radial forces to grip the clot. If the outward radial force is too low, the device loses grip with the clot an cannot remove it from the blood vessel. On the contrary, when the outward radial force is too low, the device may damage the vessel.

Therefore, it is an objective on the invention to come up with a clot removal device that is more performant in removing blood clots, while avoiding damaging the surrounding blood vessels.

### SUMMARY

To solve the aforementioned challenges, the invention relates to a clot removal device for removing at least one clot from an inside of a blood vessel, said blood vessel comprising a bifurcation wherein a proximal blood vessel is divided into at least two distal blood vessels, said distal blood vessels, said clot removal device comprising:
- a guiding wire configured for navigating the clot removal device inside said blood vessel,
- a clot removal member, configured to be positioned at the bifurcation, the clot removal member comprising:
   ∘ a base configured to be positioned inside the proximal blood vessel, the base being connected to the guiding wire, and
   ∘ two anchoring members including a first anchoring member and a second anchoring member, wherein at least one of the first anchoring member and second anchoring member is configured to be positioned inside at least one of the two distal blood vessels, the anchoring members being connected to the base, each anchoring member comprising at least one self-expanding portion configured to adopt:
      ▪ a collapsed configuration configured for navigating the clot removal device inside said blood vessel, and
      ▪ an expanded configuration configured for anchoring to the clot.

In other words, the invention is a clot removal device comprising two anchoring members capable of anchoring to the clot. Having two anchoring members allows to improve the grip on the clot and to improve the chances of removing the clot in a single intervention. The clot removal device is particularly useful to remove high fibrin rich clots, large clots and clots located in arterial bifurcations, which usually require the use of two conventional clot removal devices simultaneously introduced at the blood clot site. Indeed, using two clot removal devices requires longer surgical procedures and it is technically more difficult to navigate the two clot removal devices at the same time. The device of the invention allows the deployment of two anchoring members in a single step, with one single device, which means a faster and easier maneuver, accessible to all operators.

According to an embodiment, each anchoring member is configured to wrap around a first longitudinal axis, the self-expanding portion having:
- a collapsed diameter when in the collapsed configuration, and
- an expanded diameter when in the expanded configuration,
the expanded diameter being greater than the collapsed diameter.

In other words, the two anchoring members have a tubular shape configured to anchor to the blood clot. Each anchoring member is configured to self-expand to adapt to the size of the surrounding blood vessel.

In practice, the clot removal member is a layered structure comprising a distal region and a proximal region positioned along a third longitudinal axis of the clot removal member, the distal region comprising a longitudinal slit so as to form the first anchoring member and the second anchoring member on both sides of the slit, the base being formed in the proximal region.

Advantageously, the clot removal member is a mesh structure, wherein the dimensions of the motif of the mesh is adapted to better anchor to the blood clot.

In a preferred embodiment, the first anchoring member has a length greater than a length of the second anchoring member. The purpose of having different lengths for the anchoring members is to increase the clot removal effect into the proximal vessel, where the clot is bigger, and also, at the same time, remove the clot component located into the distal vessel thanks to the longer anchoring member.

According to another aspect, the invention also relates to a clot removal system comprising a delivery tube, said delivery tube comprising a clot removal device such as described above. The delivery tube is configured to allow the clot removal device to slidably move within the delivery tube. The self-expanding sections are in the collapsed configuration when contained in the delivery tube and the self-expanding sections are configured to adopt the expanded configuration when outside of the delivery tube.

According to another aspect, the invention relates to a method for removing at least one clot from an inside of a blood vessel using a clot removal device such as described previously. The blood vessel comprises a bifurcation wherein a proximal blood vessel is divided into at least two distal blood vessels. The method comprises the steps of:
- introducing the clot removal device inside said blood vessel so that the clot removal member is positioned at the bifurcation with the base positioned inside the proximal blood vessel and at least one of the first anchoring member and second anchoring member is positioned inside at least one of the two distal blood vessels, and
- making the self-expanding portions adopt the expanded configuration so that the anchoring members can anchor to the clot.

According to an embodiment, the method further comprises the step of navigating the clot removal device outside of the blood vessel using the guiding wire.

The invention also relates to a method for removing at least one clot from an inside of a blood vessel using a clot removal system such as described previously. The blood vessel comprises a bifurcation wherein a proximal blood vessel is divided into at least two distal blood vessels. The method comprises the steps of:
- introducing the clot removal system inside said blood vessel,
- slidably extracting the clot removing device from within said delivery tube, so that the self-expanding sections adopt the expanded configuration and the clot removal member is positioned at the bifurcation with the base positioned inside the proximal blood vessel and at least one of the first anchoring member and second anchoring member is positioned inside at least one of the two distal blood vessels.

### BRIEF DESCRIPTION OF THE DRAWINGS

**Figure 1** is a schematic longitudinal section view of a clot removal device positioned inside a blood vessel according to an embodiment of the invention,
**Figure 2** is a front view of the device from Figure 1 in the expanded configuration,
**Figure 3** is a cross-sectional view of the device from Figure 2 along a transversal axis of the proximal region,
**Figure 4** is a cross-sectional view of the device from Figure 2 along a transversal axis of the distal region,
**Figure 5** is a front view of the device from Figure 1 positioned inside a delivery tube and in the expanded configuration,
**Figure 6** is a cross-sectional view of the device from Figure 5 along an axis of the proximal region,
**Figure 7** is a cross-sectional view of the device from Figure 5 along an axis of the distal region,
**Figure 8** is front view of the device from Figure 1 in an unfolded configuration,
**Figure 9** is a graphical representation of the steps of the method for removing at least one clot from an inside of a blood vessel using a clot removal device from Figure 1, and
**Figure 10** is a graphical representation of the steps of the method for removing at least one clot from an inside of a blood vessel using a clot removal system according to the invention.

### DETAILED DESCRIPTION

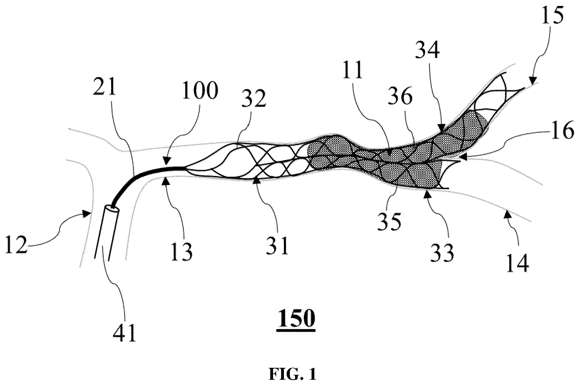

As illustrated on Figure 1, the invention relates to a clot removal system **150** that may be introduced inside a blood vessel **12,** whose blood flow is blocked by a clot **11** made of aggregated platelets reinforced by a fibrin mesh. The blood clot **11** is usually localized in a blood vessel **12** of the brain and the clot removal system **150** is designed to capture and retrieve the blood clot **11.**

The clot removal system **150** includes a delivery tube **41** configured to contain a clot removal device **100.** The clot removal device **100** comprises a clot removal member **31** configured to physically engage with the clot **11,** and a guiding wire **21,** connected to the clot removal member **31,** configured for navigating the clot removal member **31**

The guiding wire **21** is usually made of a strong and flexible material, such as stainless steel or nitinol, to withstand the forces applied during the procedure. The diameter of the guiding wire **21** typically ranges from approximately 0.3 mm to 0.5 mm. The length of the guiding wire **21** can range from around 160 cm to 180 cm or longer. This length ensures sufficient reach and maneuverability during the endovascular procedure. The guiding wire **21** may be connected to the clot removal member **31** by a weld.

The clot removal member **31** comprises a base **32** and at least two anchoring members **33, 34.** The number of anchoring members **33, 34** may be equal to two or three or more depending on the application.

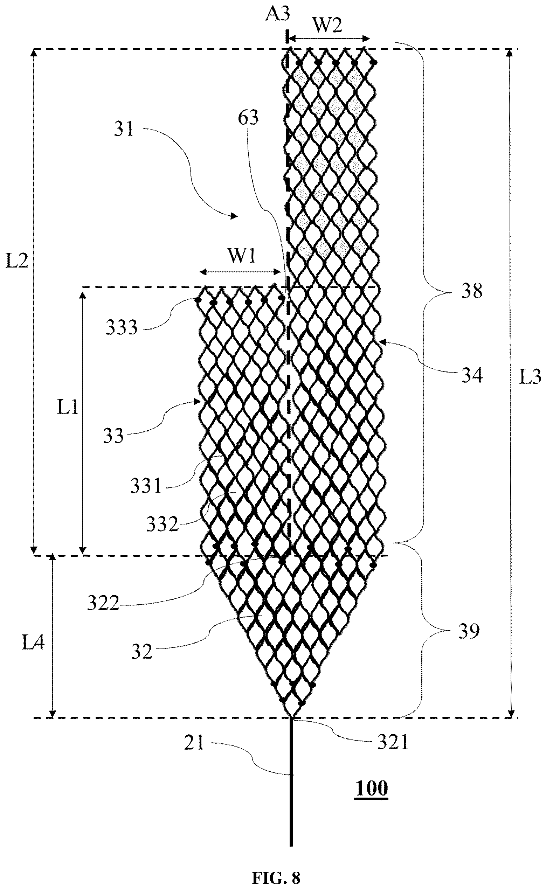

Figure 8 is a representation of a clot removal member **31** with two anchoring members **33, 34,** in a flattened configuration. The clot removal member **31** comprises a distal region **38** and a proximal region **39** positioned alongside a third longitudinal axis **A3** of the clot removal member **31.**

The base **32** is formed in the proximal region **39.** The base **32** preferably has a triangular shape with an apex **321** configured to be connected to the guiding wire **21.** The side **322** of the triangle, opposite to the apex **321** is connected to the anchoring members **33, 34.**

The distal region **38** of the clot removal member **31** has a parallelepipedal shape. A longitudinal slit **63** is formed along the third longitudinal axis **A3** of the distal region **38,** so as to form the first anchoring member **33** and the second anchoring member **34** on both sides of the slit **63.** Alternatively, several slits may be formed along the third longitudinal axis **A3** to create more than two anchoring members.

Alternatively, the clot removal member **31** may be formed using two distinct layered structures that may be joined in the proximal region **39** to form the base **32.** The layered structures may be joined by intertwining or soldering the layered structures together.

The total length **L3** of the clot removal member **31** typically ranges from about 20 mm to 70 mm, and preferably 50 mm. The base **32** preferably has a length **L4** comprised between 5 mm and 10 mm. The first anchoring member **33** and the second anchoring member **34** have lengths **L1, L2** comprised between 20 mm and 45 mm. The first anchoring member **33** and the second anchoring member **34** may have the same length **L1, L2.** Alternatively, the first anchoring member **33** and the second anchoring member **34** have different lengths **L1, L2.** For instance, the length **L1** of the first anchoring member **33** may be greater than the length **L2** of the second anchoring member **34.** As an example, the length **L2** of the second anchoring member **34** may be comprised between 35 mm and 45 mm and the length **L1** of the first anchoring member **33** may be comprised between 25 mm and 35 mm. Advantageously, the length **L2** is greater of 15 mm to 25 mm, preferably 20 mm, than the length **L1.**

The side **322** of the triangle opposite to the apex **321** may have a width comprised between 8 mm and 15 mm. The width **W1** of the first anchoring member **33** may be comprised between 5 mm and 10 mm and the width **W2** of the second anchoring member **34** may be comprised between 5 mm and 10 mm. Preferably, the first anchoring member **33** and the second anchoring member **34** have the same width **W1, W2.**

The clot removal member **31** is preferably a mesh structure made of interconnected wires **331.** The motif of the mesh may for instance be a square, diamond, or circular motif **332** or a combination thereof. The motif **332** is designed to optimize clot **11** engagement, capture, and retrieval efficiency. The spacing and orientation of the wires **331** within the motif **332** is crucial for ensuring proper clot **11** entanglement and minimizing the risk of clot **11** migration during retrieval. For example, the spacing between the motifs **332** may range from a fraction of a millimeter to a few millimeters. The individual wires **331** in the mesh structure can vary based on the clot removal member **31** design. The thickness of the wires **331** can range from a fraction of a millimeter to a few millimeters. The density of the mesh structure refers to the spacing between the individual wires **331.** The mesh density can range from 0.1 mm to 0.5 mm.

The clot removal member **31** is made of a biocompatible metal alloy. The most commonly used material is nitinol, a nickel-titanium alloy. Nitinol is known for its excellent shape-memory characteristics, allowing the clot removal member **31** to be compressed for delivery and then expand to its pre-shaped form upon deployment. Nitinol also provides flexibility, strength, and good radial force to engage and capture the clot **11** effectively. The biocompatibility of nitinol ensures that the clot removal member **31** is well-tolerated by the body, thus minimizing the risk of adverse reactions or complications.

Advantageously, radiopaque markers **333,** which are small metallic components, may be incorporated into the clot removal member **31** and more precisely, in the proximal region **39** and at the distal end of the distal region **38** and in the central part of the clot removal member **31,** where the base **32** connects to the first anchoring member **33** and the second anchoring member **34.** These radiopaque markers **333** are visible under X-ray fluoroscopy and allow to track and guide the positioning of the clot removal device **100** within the blood vessel **12.** The radiopaque markers **333** help ensure accurate placement and retrieval of the clot removal device **100.** The radiopaque markers **333** are usually made of materials such as platinum, tantalum or tungsten or other radiodense materials.

The clot removal member **31** may advantageously be obtained from a larger mesh and cut to the correct shape and dimensions using laser cutting.

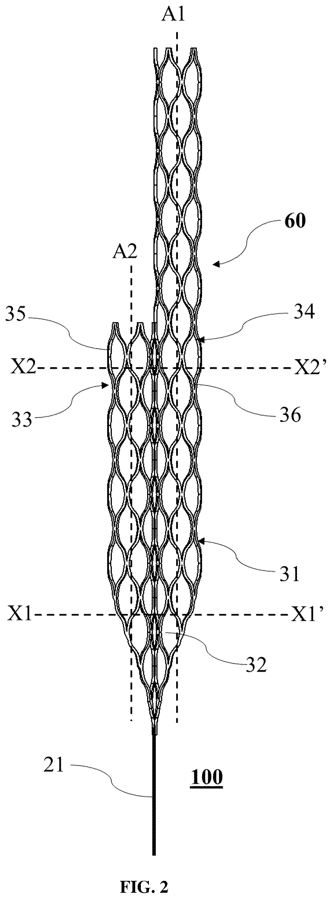

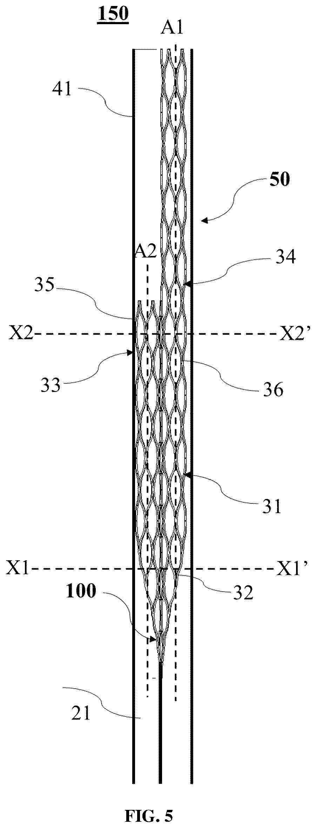

The clot removal member **31** is configured to adopt two configurations: a collapsed configuration **50,** illustrated in Figure 5, for navigating the clot removal device **100** inside the body, and an expanded configuration **60,** illustrated in Figure 2, for anchoring to the clot **11.**

In these two configurations, the clot removal member **31** is folded around two longitudinal axes, including a first longitudinal axis **A1** and a second longitudinal axis **A2.**

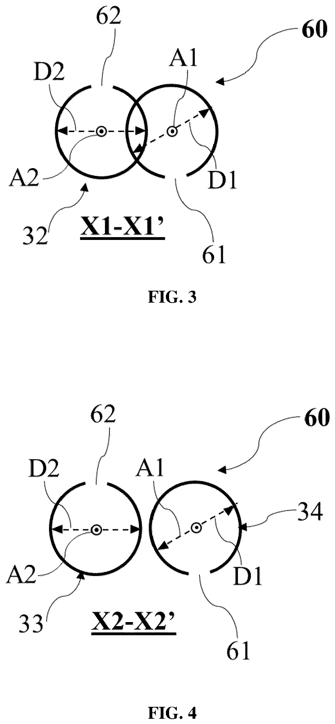

As illustrated in Figure 4, in the expanded configuration **60,** the first anchoring member **33** is folded around the first longitudinal axis **A1** and the second anchoring member **34** is folded around the second longitudinal axis **A2.** Both anchoring members **33, 34** thus adopt a tubular shape with an opened distal end. Therefore, the cross-section of the anchoring members **33, 34** alongside the **X2-X2'** axis is an opened circle comprising a slit **61, 62.** The diameter **D1, D2** of the anchoring members **33, 34** in the expanded configuration **60** can range from approximately 2 mm to 6 mm. This expanded configuration **60** allows the anchoring members **33, 34** to create a scaffold that encompasses the clot **11,** thus enabling effective entanglement and capture.

The base **32** may be formed by entwining the tubes formed by the anchoring members **33, 34** such as illustrated on Figure 3. Therefore, the cross-section of the base **32** alongside the **X1-X1'** axis comprises two entwined opened circles comprising each a slit **61, 62.** Alternatively, when the base **32** is formed using two distinct layered structures joined in the proximal region **39,** the base **32** folds around a single base axis, while the first anchoring member **33** is folded around the first longitudinal axis **A1** and the second anchoring member **34** is folded around the second longitudinal axis **A2.**

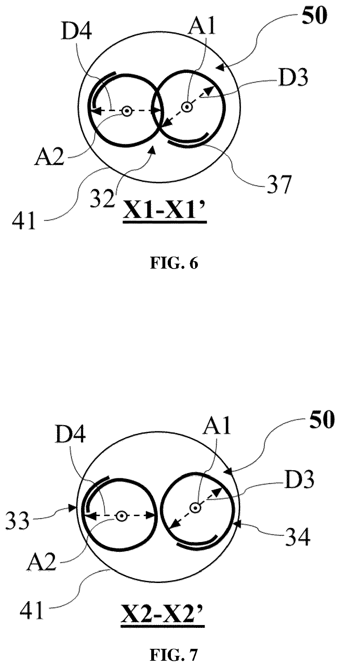

As illustrated in Figure 7, in the collapsed configuration **50,** the first anchoring member **33** is folded around the first longitudinal axis **A1** and the second anchoring member **34** is folded around the second longitudinal axis **A2.** Both anchoring members **33, 34** also adopt a tubular shape with an opened distal end. Therefore, the cross-section of the anchoring members **33, 34** alongside the **X2-X2'** axis is a circle comprising a slit **61, 62,** wherein the extremities of the circle bordering the slit **61, 62** are overlapping. The diameter **D3, D4** of the anchoring members **33, 34** in the collapsed configuration **50** is therefore smaller than in the expanded configuration and can range from approximately 1 mm to 4 mm. This collapsed configuration allows the anchoring members **33, 34** to be contained into the delivery tube **41.**

The base **32** is also formed by entwining the tubes formed by the anchoring members **33, 34** such as illustrated on Figure 6. Therefore, the cross-section of the base **32** alongside the **X1-X1'** axis comprises two entwined opened circles comprising each a slit **61, 62** and wherein the extremities of the circle bordering the slit **61, 62** are overlapping.

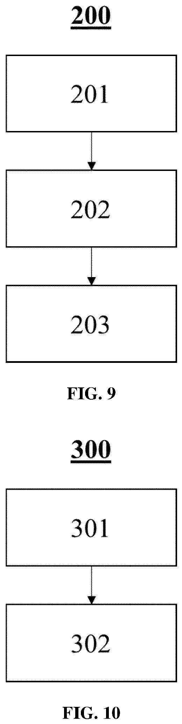

As illustrated in Figure 9, the invention also relates to a method **200** for removing at least one clot **11** from an inside of a blood vessel **12** using a clot removal device **100** such as described above. The clot removal device **100** may be introduced **201** inside a blood vessel **12** such as illustrated in Figure 1, so that the clot removal member **31** is positioned at the bifurcation **16** of the blood vessel **12.** Preferably, the base **32** and the first anchoring member **33** are positioned inside the proximal blood vessel **13,** while the second anchoring member **34** extends inside one distal blood vessel **14, 15.** The self-expanding portions **35, 36** of the first anchoring member **33** and second anchoring member **34** are then configured to adopt the expanded configuration **60** to anchor to the clot **11.** After being anchored to the clot **11,** the clot removal device **100** is navigated **203** outside of the blood vessel **12** using the guiding wire **21.**

The invention also relates to a method **300** for removing at least one clot **11** from an inside of the body using a clot removal system **150** including a clot removal device **100.** The removal of the clot **11** is performed through an endovascular surgery procedure, which is a minimally invasive surgery involving the selection of an arterial access point, established depending on the localization of the clot **11.** The arterial access point may for instance be localized in the femoral artery.

Usually, a guiding catheter is first threaded through the arterial system, for instance under X-ray guidance, to reach the targeted blood vessel **12** where the clot **11** is located. The guiding catheter serves as a pathway for the subsequent insertion of the clot removal system **150.** The specific dimensions and material used for the guiding catheter depend on the clinical scenario, patient anatomy, and the preferences of the surgeon. The guiding catheter may have an inner diameter adapted to receive the clot removal system **150,** for instance comprised between 1 mm and 3mm and an outer diameter comprised between 1.5 mm and 3;5 mm. Such guiding catheters are preferably made of biocompatible materials, such as polyethylene or polyurethane, that provide flexibility, strength, and radiopacity. The choice of material aims to optimize maneuverability, durability, and visibility during the surgery.

The clot removal system **150** is then introduced **301** inside the body, either in a single step wherein the clot removal device **100** is directly contained in the delivery tube **41** or in two consecutive steps such as described below.

The delivery tube **41** is navigated through the guiding catheter to reach the precise location of the clot **11.** This step requires skill and expertise to ensure proper positioning. Once the delivery tube **41** is in place near the clot **11,** the clot removal device **100** is introduced through the delivery tube **41** and guided to the clot **11** site using the guiding wire **21**

The dimensions of the delivery tube **41** are adapted to allow optimal navigation inside the guiding catheter. Moreover, the dimensions are also chosen depending on the target blood vessel's size and the compatibility with the clot removal device **100** used. Preferably, the delivery tube **41** has an outer diameter ranging from 0.3 mm to 0.7 mm. The inner diameter is preferably comprised between 0.2 mm and 0.5 mm. Delivery tubes **41** are typically made of flexible and biocompatible materials, such as polyurethane or polyethylene. These materials provide good trackability, torque control, and pushability while navigating through tortuous blood vessels **12.**

In a subsequent step, the clot removal member **31** of the clot removal device **100** is deployed from the collapsed configuration **50** to the expanded configuration **60** within the blood vessel **12,** allowing the two anchoring members **33, 34** to anchor to the clot **11.** The clot removal member **31** may be deployed automatically when being slidably extracted **302** from within the delivery tube **41.** The self-expending portions **35, 36** are no longer compressed by the delivery tube **41** and can go back to their original expanded configuration **60.**

The delivery tube **41** together with the expanded clot removal device **100** is then slowly pulled back. This action entangles the clot **11** within the clot removal member **31** mesh structure, thus facilitating its capture and removal from the blood vessel **12.** Once the clot **11** is safely captured within the clot removal member **31,** the entire system **150,** including the clot removal device **100** and the delivery tube **41,** is carefully withdrawn from the body, thus restoring blood flow in the affected blood vessel **12.**

The procedure may be guided by fluoroscopy, a real-time X-ray imaging technique that helps visualize the blood vessels and guide the placement and retrieval of the clot removal device **100.**

## Claims

1. A clot removal device (100) for removing at least one clot (11) from an inside of a blood vessel (12), said blood vessel (12) comprising a bifurcation (16) wherein a proximal blood vessel (13) is divided into at least two distal blood vessels (14, 15), said clot removal device (100) comprising:
- a guiding wire (21) configured for navigating the clot removal device (100) inside said blood vessel (12),
- a clot removal member (31), configured to be positioned at the bifurcation (16), the clot removal member (31) comprising:
∘ a base (32) configured to be positioned inside the proximal blood vessel (13), the base (32) being connected to the guiding wire (21), and
∘ two anchoring members (33, 34), including a first anchoring member (33) and a second anchoring member (34), wherein at least one of the first anchoring member (33) and second anchoring member (34) is configured to be positioned inside at least one of the two distal blood vessels (14, 15), the anchoring members (33, 34) being connected to the base (32), each anchoring member (33, 34) comprising at least one self-expanding portion (35, 36) configured to adopt:
▪ a collapsed configuration (50) configured for navigating the clot removal device (100) inside said blood vessel (12), and
▪ an expanded configuration (60) configured for anchoring to the clot (11).

2. The device according to claim **1, *wherein*** each anchoring member (33, 34) is configured to wrap around a first longitudinal axis (A1, A2), the self-expanding portion (35, 36) having:
- a collapsed diameter (D3, D4) when in the collapsed configuration (50), and
- an expanded diameter (D1, D2) when in the expanded configuration (60),
the expanded diameter (D1, D2) being greater than the collapsed diameter (D3, D4).

3. The device according to claim **1** or **2, *wherein*** the clot removal member (31) is a layered structure comprising a distal region (38) and a proximal region (39) positioned along a third longitudinal axis (A3) of the clot removal member (31), the distal region (38) comprising a longitudinal slit (63) so as to form the first anchoring member (33) and the second anchoring member (34) on both sides of the slit (63), the base (32) being formed in the proximal region (39).

4. The device according to any of claims **1** to **3, *wherein*** the clot removal member (31) is a mesh structure.

5. The device according to any of claims **1** to **4, *wherein*** the first anchoring member (33) has a length (L1) greater than a length (L2) of the second anchoring member (34).

6. The device according to any of claims **1** to **5, *wherein*** the first anchoring member (33) is configured to be positioned inside one of the two distal blood vessels 14, 15) and the second anchoring member (34) is configured to be positioned inside the proximal blood vessel (13).

7. A clot removal system (150) comprising a delivery tube (41), said delivery tube (41) comprising a clot removal device (100) according to any of claim **1** to **6,** said delivery tube (41) being configured to allow the clot removal device (100) to slidably move within said delivery tube (41), wherein the self-expanding portion (35, 36) are in the collapsed configuration (50) when contained in the delivery tube (41) and wherein the self-expanding portion (35, 36) are configured to adopt the expanded configuration (60) when outside of the delivery tube (41).
